(19) Europäisches Patentamt
European Patent Office
Office européen des brevets

(11) **EP 3 674 956 A1**

(12) **EUROPEAN PATENT APPLICATION**

(43) Date of publication:
**01.07.2020 Bulletin 2020/27**

(51) Int Cl.:
**G06F 30/10** $^{(2020.01)}$        **G06F 30/17** $^{(2020.01)}$
**G06F 30/20** $^{(2020.01)}$

(21) Application number: **19211978.2**

(22) Date of filing: **28.11.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2018 US 201816235424**

(71) Applicant: **Dassault Systemes Simulia Corp.**
**Johnston, RI 02919 (US)**

(72) Inventors:
• **COJOCARU, Dan**
  **Johnston, Rhode Island 02919 (US)**
• **WOHLEVER, James Christopher**
  **Johnston, Rhode Island 02919 (US)**
• **DALRYMPLE, Tod Olan**
  **Johnston, Rhode Island 02919 (US)**

(74) Representative: **Müller-Boré & Partner**
**Patentanwälte PartG mbB**
**Friedenheimer Brücke 21**
**80639 München (DE)**

(54) **SYSTEM AND METHOD FOR STABILITY-BASED CONSTRAINED NUMERICAL CALIBRATION OF MATERIAL MODELS**

(57)    A computer simulation system is configured to display to a user a graphical user interface to allow the user to import experimental test data, identify a material model that includes one or more parameters to be calibrated during a material model calibration process, perform an iterative optimization process to calibrate the material model, the iterative optimization process uses an optimization algorithm that enforces a constraint based on Drucker's stability criterion across one or more predetermined strain ranges to generate a calibrated material model, assign the calibrated material model to a component of a simulation model based on input from the user, a real-world equivalent of the component being made of the physical material, and perform a simulation that includes the component, the simulation using the stable material model and stable set of parameters to simulate response of the real-world equivalent during the simulation.

FIG. 3

**Description**

BACKGROUND

This disclosure relates generally to simulation systems and, more particularly, to systems and methods for stability-based constrained numerical calibration of material models in computer simulations.

Material constitutive models are mathematical models which attempt to replicate the behavior of a real material in a computer simulation software system. Such material models represent a significant aspect of the simulation software systems. A material constitutive model may be used to simulate a simple component involving the material, such as a component of a vehicle chassis, or a more complex assembly such as an entire vehicle chassis.

Some known systems use experimental data derived from real-world tests to generate a material constitutive model for a particular material. Real materials are commonly tested in a laboratory under specific loading conditions. During such tests, a specimen made of the real material is subjected to one or more deformation modes, such as uniaxial deformation, biaxial deformation, planar deformation, simple shear deformation, or volumetric deformation, where deformation can be in tension or compression. During these testing conditions, the response of the real material may be recorded as a sequence of pairs of strain and stress values, representing experimental strain - stress data sets. Such experimentally collected data sets are typically not directly used in numerical simulations. Rather, a particular mathematical material model is selected for use in the simulation, and the experimental strain - stress data sets are used to calibrate material parameters of the mathematical material model such as to approximately numerically replicate the experimental data. One common approach is to start with an initial guess for the parameters of the mathematical constitutive response and iterate to minimize the errors between the simulated response and the corresponding experimental data. Some systems may use numerical optimization algorithms to iteratively minimize the errors between the model and the experimental data.

However, such known simulation software systems may generate an unstable material model. For example, a material model for a rubber band may complete successfully for a deformation of the rubber band at twice its natural length, but that model may be unstable for deformations beyond three times the rubber band's length. When a user (e.g., an engineer, scientist) executes a component simulation that uses the unstable material model in the simulation software system, the simulation may not succeed (e.g., may not run to completion). The user may be left wondering why the simulation did not succeed, requiring the user to investigate various failure potentials before potentially discovering that the unstable material model caused the failure. Even if the user is informed that the material model is unstable, that information does not necessarily implicate the material model in larger simulations, and also does not provide a solution, just one clue as to what may be causing the failure. What is needed is a system and method for providing stable material constitutive models for the simulation software system.

BRIEF DESCRIPTION

In one aspect, a computer simulation system is provided. The computer simulation system includes a memory storing an experimental test data set. The experimental test data set includes experimentally-obtained stress-strain data for a sample of a physical material subjected to one or more deformation modes during a real-world experiment. The computer simulation system also includes a processor configured to execute instructions stored in the memory. When executed by the processor, the instructions cause the processor to at least display to a user a graphical user interface configured to allow the user to import the experimental test data set. The instructions also cause the processor to receive an identification of the test data set for material model calibration. The instructions further cause the processor to identify a material model. The material model includes one or more parameters of a parameter set to be calibrated during the material model calibration. The parameter set starts with a set of initial parameter values. The instructions also cause the processor to perform an iterative optimization process to calibrate the material model. The iterative optimization process uses an optimization algorithm that enforces a constraint based on Drucker's stability criterion across one or more predetermined strain ranges. The instructions further cause the processor to terminate the iterative optimization process. The optimization process generates a calibrated material model. The instructions also cause the processor to assign the calibrated material model to a component of a simulation model based on input from the user. A real-world equivalent of the component is made of the physical material. The instructions further cause the processor to perform a simulation that includes the component. The simulation uses the stable material model and stable set of parameters to simulate response of the real-world equivalent during the simulation.

In another aspect, a method of calibrating a material model for use in a computer simulation is provided. The method is performed by a processor with a memory. The method includes storing, in the memory, an experimental test data set. The experimental test data set includes experimentally-obtained stress-strain data for a sample of a physical material subjected to one or more deformation modes during a real-world experiment. The method also includes displaying to a user a graphical user interface configured to allow the user to import the experimental test data set. The method also includes receiving an identification of the test data set for material model calibration. The method further includes identifying a material model. The material model includes one or more parameters of a parameter set to be calibrated during the material model calibration. The parameter set starts with a set of initial parameter values. The method also includes performing an iterative optimization process to calibrate the material model. The iterative optimization process uses an optimization algorithm that enforces a constraint based on Drucker's stability criterion across one or more predetermined strain ranges. The method further includes terminating the iterative process. The iterative process generates a calibrated material model. The method also includes assigning the calibrated material model to a component of a simulation model based on input from the user. A real-world equivalent of the component is made of the physical material. The method further includes performing a simulation that includes the component. The simulation uses the stable material model and stable set of parameters to simulate response of the real-world equivalent during the simulation.

In yet another aspect, a computer-readable storage media having computer-executable instructions embodied thereon. When executed by at least one processor, the computer-executable instructions cause the processor to store, in the memory, an experimental test data set. The experimental test data set includes experimentally-obtained stress-strain data for a sample of a physical material subjected to one or more deformation modes during a real-world experiment. The computer-executable instructions also cause the processor to display to a user a graphical user interface configured to allow the user to import the experimental test data set. The computer-executable instructions further cause the processor to receive an identification of the test data set for material model calibration. The computer-executable instructions also cause the processor to identify a material model. The material model includes one or more parameters of a parameter set to be calibrated during the material model calibration. The parameter set starts with a set of initial parameter values. The computer-executable instructions further cause the processor to perform an iterative optimization process to calibrate the material model. The iterative optimization process uses an optimization algorithm that enforces a constraint based on Drucker's stability criterion across one or more predetermined strain ranges. The computer-executable instructions also cause the processor to terminate the iterative process. The iterative process generates a calibrated material model. The computer-executable instructions further cause the processor to assign the calibrated material model to a component of a simulation model based on input from the user. A real-world equivalent of the component is made of the physical material. The computer-executable instructions also cause the processor to perform a simulation that includes the component. The simulation uses the stable material model and stable set of parameters to simulate response of the real-world equivalent during the simulation.

BRIEF DESCRIPTION OF THE DRAWINGS

FIGS. 1-8 show exemplary embodiments of the methods and systems described herein.

FIG. 1 is a diagram of an example simulation system that includes a material calibration module for generating material models using stability-based constrained numerical calibration.

FIG. 2 is a diagram illustrating various sub-modules of the material calibration module shown in FIG. 1.

FIG. 3 is a flow chart of an example calibration process for generating material models using stability-based constrained numerical calibration.

FIG. 4 is an illustration of an example view of a graphical user interface provided by the material calibration module during a calibration process without stability constraint.

FIG. 5 is an illustration of another example view of the graphical user interface shown in FIG. 4 that includes stability indicators panel provided by the material calibration module during the unconstrained calibration process.

FIG. 6 is an illustration of an example view of a graphical user interface provided by the material calibration module during a calibration process that includes stability constraint consideration as described herein.

FIG. 7 illustrates a view of the example shown in FIG. 6 after the user 102 has executed the stability-constrained calibration process.

FIG. 8 is an illustration of another example view of the graphical user interface shown in FIG. 7 that includes the stability indicators panel after the stability-constrained calibration process.

DETAILED DESCRIPTION

The following detailed description illustrates embodiments of the disclosure by way of example and not by way of limitation. It is contemplated that the disclosure has general application to computer simulation with material models.

A simulation system provides a material calibration module and associated methods that, as described herein, determine a stable material model for use in simulating a particular real-world material. During a material model calibration process, the material calibration module determines parameter values for a base material constitutive model such that the resulting material model ("calibrated material model") is configured to be stable across certain strain ranges.

During configuration, the material calibration module provides a graphical user interface ("GUI") through which a user (e.g., an engineer, a product tester) provides a set of material test data (e.g., strain-stress test data for one or more deformation modes) from a real-world lab test of the material to the material calibration module. Further, the user also identifies a particular base material model from a list of available material models provided by the simulation system. The selected material model, once calibrated, may be used during future simulations involving the associated material. The selected base material model includes one or more model parameters that, once calibrated, cause the material model to approximate the associated material during simulation. The user may select which of the parameters to use during the calibration process, and may alter starting values (e.g., coefficients) of each of the selected parameters. Further, if the user activates stability control for the calibration, the user may then identify one or more strain range settings for various deformations of interest (e.g., lower and upper thresholds for nominal strain, volume ratio, and shear strain) over which the user wishes the material model to be stable. The user then initiates calibration of the material model.

During calibration, the material calibration module determines calibrated values for each of the selected parameters of the material model such that the resultant calibrated material model is stable across the identified strain ranges. More specifically, the material calibration module iteratively uses a numerical constrained optimization algorithm to minimize an objective function that incorporates the errors between the test data set and the predicted response of the material model at the present iteration (e.g., with the present coefficients), together with constraints derived from one or more stability criteria. The optimization algorithm enforces the constraints of the stability criteria during a minimization process, iteratively converging on a set of material parameters that both satisfies the stability criteria over the identified strain ranges and minimizes the errors between the test data set and the corresponding response predicted by the material model. Upon convergence, the final parameter values coupled with the material model represent the resultant calibrated material model.

During simulation (e.g., finite element simulation), the simulation system uses the calibrated material model, for example, to generate a series of linearized algebraic equations which represent the stiffness of the component being simulated. Using unstable material responses in a numerical finite element simulation can lead to non-unique or unphysical solutions, or to a break-down of the solution process. As such, the simulation system may use the calibrated material model to model components involving the underlying material. A simulation (e.g., a Finite Element based simulation) typically contains various ingredients (e.g., geometry, mesh, material models, loads, boundary conditions, and so forth). A mesh is typically made of a set of finite elements (e.g., tetrahedrons, hexahedrons, pyramids, triangles, and so forth) that represent the geometry of a part or assembly in the simulation. Each finite element in the simulation is assigned a material model that describes how that element behaves under applied loads. The simulation system may allow the user to assign the calibrated material model to various mesh regions (e.g., sets of finite elements) in a simulation model. As such, the simulation system may use that calibrated material model when simulating those mesh regions during an active simulation.

As used herein, an element or step recited in the singular and proceeded with the word "a" or "an" should be understood as not excluding plural elements or steps, unless such exclusion is explicitly recited. Furthermore, references to "exemplary embodiment" or "one embodiment" of the present disclosure are not intended to be interpreted as excluding the existence of additional embodiments that also incorporate the recited features.

As used herein, the term "database" may refer to either a body of data, a relational database management system (RDBMS), or both. As used herein, a database may include any collection of data including hierarchical databases, relational databases, flat file databases, object-relational databases, object oriented databases, and any other structured collection of records or data that is stored in a computer system. The above examples are example only, and thus are not intended to limit in any way the definition and/or meaning of the term database. Examples of RDBMS's include, but are not limited to including, Oracle® Database, MySQL, IBM® DB2, Microsoft® SQL Server, Sybase®, and PostgreSQL. However, any database may be used that enables the systems and methods described herein. (Oracle is a registered trademark of Oracle Corporation, Redwood Shores, California; IBM is a registered trademark of International Business Machines Corporation, Armonk, New York; Microsoft is a registered trademark of Microsoft Corporation, Redmond, Washington; and Sybase is a registered trademark of Sybase, Dublin, California.)

As used herein, a processor may include any programmable system including systems using micro-controllers, reduced instruction set circuits (RISC), application specific integrated circuits (ASICs), logic circuits, and any other circuit or processor capable of executing the functions described herein. The above examples are example only, and are thus not intended to limit in any way the definition and/or meaning of the term "processor."

As used herein, the terms "software" and "firmware" are interchangeable, and include any computer program stored in memory for execution by a processor, including RAM memory, ROM memory, EPROM memory, EEPROM memory, and non-volatile RAM (NVRAM) memory. The above memory types are example only, and are thus not limiting as to the types of memory usable for storage of a computer program.

In one embodiment of the present disclosure, a computer program is provided, and the program is provided on a computer readable medium. In an exemplary embodiment, the system is executed on a single computer system, without requiring a connection to a server computer. In a further embodiment, the system is being run in a Windows® environment (Windows is a registered trademark of Microsoft Corporation, Redmond, Washington). In yet another embodiment, the system is run on a mainframe environment and a UNIX® server environment (UNIX is a registered trademark of X/Open Company Limited located in Reading, Berkshire, United Kingdom). The application is flexible and designed to run in various different environments without compromising any major functionality. In some embodiments, the system includes multiple components distributed among a plurality of computing devices. One or more components may be in the form of computer-executable instructions embodied in a computer-readable medium. The systems and processes are not limited to the specific embodiments described herein. In addition, components of each system and each process can be practiced independent and separate from other components and processes described herein. Each component and process can also be used in combination with other assembly packages and processes.

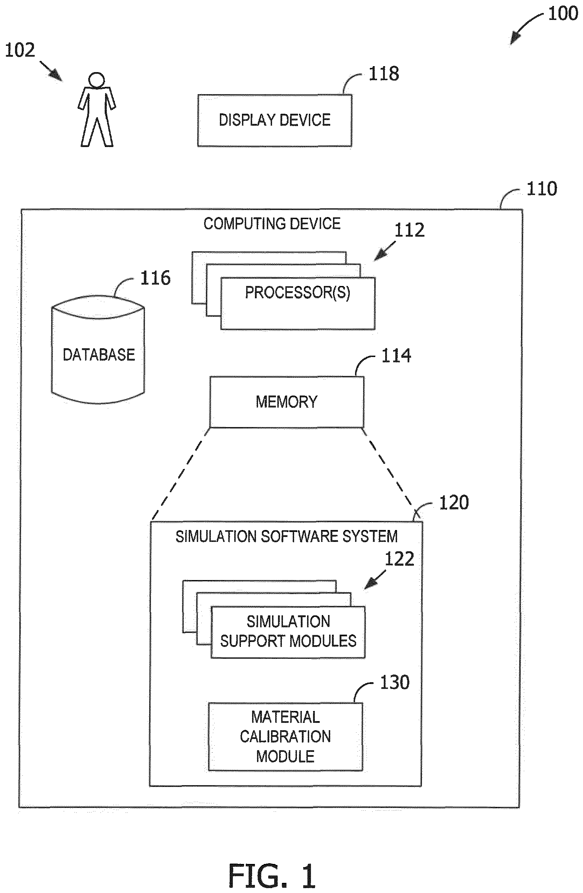

FIG. 1 is a diagram of an example simulation system 100 that includes a material calibration module 130 for generating material models using stability-based constrained numerical calibration. In the example embodiment, the simulation system 100 includes a computing device 110 executing a simulation software system 120. The computing device 110 includes one or more processors 112 and a memory 114 storing the simulation software system 120. The computing device 110 is coupled to a display device 118 that allows a user 102 (e.g., an engineer, a scientist, a product developer) to interface with the simulation software system 120 to perform various simulation activities, such as executing simulations on assemblies or components or calibrating material models used in such simulations, as described herein. The computing device 110 may include other conventional hardware and software components of a conventional computing device (e.g., communication devices such as network interface cards or input/output devices such as keyboard, pointing device, touch screen, audio input/output devices, and such), but that these devices are not shown for purposes of brevity.

The simulation software system 120 includes various simulation support modules 122 that may be used to prepare for, execute, and evaluate the results of computer-based simulations. Such simulation support modules 122 may include, for example, 3D product design tools such as a computer-aided drafting (CAD) module, and analysis tools such as a finite element analysis module, a computational fluid dynamics module, and a computational electromagnetics module supporting computer-aided engineering (not separately shown). The simulation software system 120 allows users 102 to configure and execute various types of computer-implemented simulations, for example, in an effort to understand how their real-world counterparts will act or react under various conditions.

The simulation software system 120, in the example embodiment, also includes the material calibration module 130. The material calibration module 130 allows the user 102 to calibrate a material constitutive model (or just "material model") (not depicted in FIG. 1) to approximate properties of a subject material based on real-world test results. Material constitutive models are mathematical models which attempt to replicate the behavior of real materials under various modes of deformation. Many material constitutive models are known in the art, each of which may include one or more parameters that can be configured to alter the responses of the material model.

To prepare for calibration of the material model within the simulation software system 120, the user 102 gathers real-world test data. A real material (also referred to herein as the "subject material") may be tested in a laboratory under specific loading conditions, during which a specimen made of the real material is subjected to one or more deformation modes (e.g., tensile and compressive uniaxial loading, tensile and compressive biaxial loading, tensile and compressive planar loading (also known as pure shear loading), volumetric loading, and simple shear loading). During these testing conditions, the responses of the real material are recorded, by experimental equipment, as a sequence of pairs of strain and stress values ($\varepsilon$, $T$) ("strain-stress data"), and in some instances may also include time. In some embodiments, the test data may be stored in memory 114 or in a database 116, either locally as shown in FIG. 1, or remotely on another computing device. For example, the subject material may be an elastomer that is planned for use in a bushing for an engine mount of an automobile. As such, a specimen made of that elastomer may be tested in the lab to capture strain-stress data under various modes of deformation.

However, these experimentally obtained strain-stress data sets may not be directly usable in numerical simulations. Rather, a material model is selected for use in simulating the subject material, and that material model is then calibrated to approximate the subject material. Some material models may be better suited to approximate the subject material. In some embodiments, the user 102 may select the material model from a list of available material models (e.g., hyperelastic, hyperfoam, and so forth). Each material model includes one or more configurable parameters (e.g., typically coefficients of underlying equation variables) through which the response of the material model may be calibrated to alter the model response. Once a particular "base" material model (e.g., an uncalibrated material model) is selected, the base material model is seeded with initial parameter values (e.g., default values, user-provided values). The material calibration module 130 then alters one or more of the parameters over a sequence of iterations, minimizing error between the experimental data and the simulated response of the model at each iteration.

In the example embodiment, the material calibration module 130 executes a numerical optimization algorithm that enforces stability constraints during the minimization process, iteratively converging to a set of material parameters that satisfy one or more stability criteria (e.g., Drucker's stability criterion) at prescribed strain ranges while minimizing the errors between the experimental data and the model response. For a human, the task of manually manipulating parameters in such a way that simultaneously ensures both a stable response and accuracy in the model response is intractable, even for models with only a few parameters. As such, engineers are often faced with the choice of either using a material model that is unstable in certain deformation regimes or settling for non-optimal material parameters. Here, the material calibration module 130 automatically provides calibrated parameters for the material model that correspond to a stable material response for strain ranges of interest to the user 102 (e.g., strain ranges expected to occur in future simulations in which the material model will be used). This can save significant time compared to known methods which rely on manually and repeatedly altering the input test data or changing the material model.

Once generated, the calibrated material model for the subject material (e.g., the material model in conjunction with the determined parameter values) may be used in a simulation involving that material. For example, the simulation software system 120 may execute a computer-aided simulation involving the engine mount bushing made of the subject material to analyze the performance of the bushing under various conditions (e.g., supporting engine weight, chassis deformation during operation, vibration analysis, and so forth). Calibrated material models may be saved within the simulation system 100 (e.g., within database 116) and used during execution of simulations within the simulation system or exported for use in other simulation systems 100. A simulation (e.g., a Finite Element based simulation) typically contains various ingredients (e.g., geometry, mesh, material models, loads, boundary conditions, and such). The calibrated material model can, as such, be assigned to a particular finite element within the mesh. For example, the user 102 may search the database 116 for this or other material models and chose the calibrated material model of interest for particular mesh regions in the simulation model.

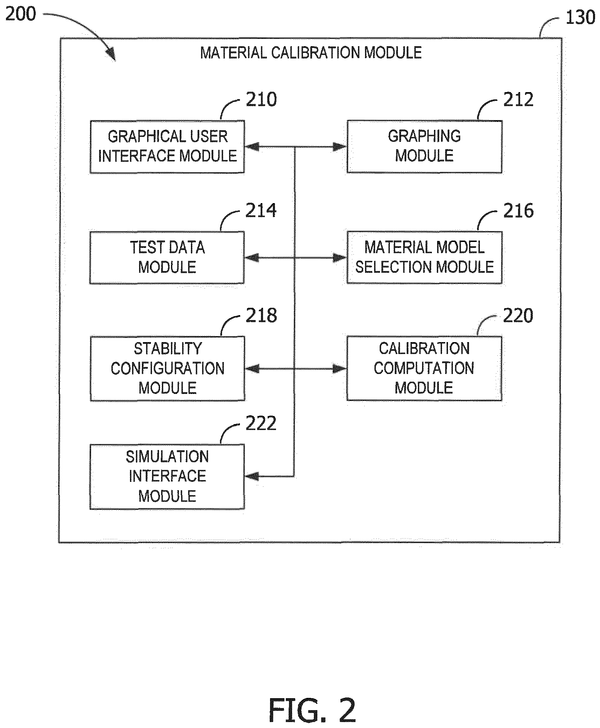

FIG. 2 is a diagram illustrating various sub-modules 200 of the material calibration module 130 shown in FIG. 1. Each of the various sub-modules of the material calibration module 130 perform various functionality for generating material models using stability-based constrained numerical calibration, as described herein. In the example embodiment, the material calibration module 130 includes a graphical user interface (GUI) module 210, a graphing module 212, a test data module 214, a material model selection module 216, a stability configuration module 218, a calibration computation module 220, and a simulation interface module 222 (collectively, "sub-modules 200"). Each of the various sub-modules 200 may interact with other sub-modules 200 when performing their respective functions.

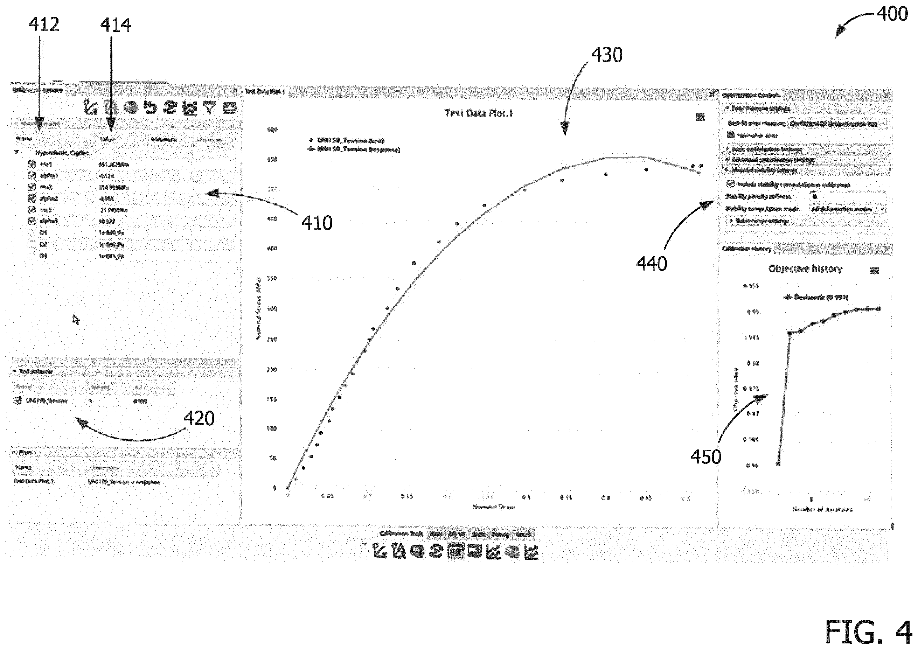

In the example embodiment, the material calibration module 130 provides a graphical user interface (not shown in FIG. 2) through which the user 102 may interface with aspects of the material calibration module 130. The GUI module 210 provides the graphical user interface functionality to the user 102 via the display device 118 for many of the sub-modules 200, allowing the user 102 to, for example, view and input configuration parameters via the material model selection module 216, provide test data via the test data module 214, view graphs prepared by the graphing module 212, configure stability settings for material calibration via the stability configuration module 218, and view results provided by the calibration computation module 220. Examples of such GUI components are shown in FIGs. 4-8.

The graphing module 212, in the example embodiment, generates and displays graphs provided by the material calibration module 130. In some embodiments, the graphing module 212 plots axes and data points from experimental data sets (or simply "test data") for a subject material (e.g., strain and stress axes, data points from the test data). Such plots allow the user 102 to, for example, visualize the test data, confirm the test data, and compare the test data to results of a material model (e.g., base, calibrated, or otherwise). In some embodiments, the graphing module 212 provides multiple plots overlaid onto a single graph (e.g., multiple sets of test data, both test data and results data). Plotting both test data and results data together may allow the user 102 to visually verify the results of the calibrated material model relative to the test data.

The test data module 214, in the example embodiment, facilitates reading of the experimental data sets and preparing the experimental data sets for use by the material calibration module 130. The test data module 214, in some embodiments, allows the user 102 to identify a source of experimental data (e.g., from a raw data source such as an excel spreadsheet, the database 116, and such). Test data may include stress and strain pairs, and in some situations may also include time. The test data module 214 may display data from the raw data file and allow the user 102 to select which data to use for calibrating the material model. The test data module 214 may also allow the user 102 to identify test parameters for the test data, such as what type of deformation the test data set represents.

The material model selection module 216, in the example embodiment, allows the user 102 to view and select, from a list of base material models, which material model to use during a calibration process for the subject material. Various material models are known. For example, for hyperelastic materials, the list of base material models may include Arruda-Boyce, Mooney-Rivlin, Neo Hooke, Ogden, Polynomial, Reduced Polynomial, Yeoh, and so forth. The material model selection module 216 may allow the user 102 to select which type of models they wish to select from, such as hyperelastic only, hyperelastic combined with Mullins effect, hyperelastic combined with viscoelasticity, or hyperelastic combined with viscoelasticity and Mullins effect. Upon selection of a particular type, the material model selection module 216 displays only material models of the selected type to the user 102.

Each material model may include one or more model parameters that may be configured by the user 102. Upon selection of a particular base material model, the material model selection module 216, in the example embodiment, displays the available model parameters to the user 102. In some embodiments, the user 102 may select which of the available model parameters for that base model to allow to be altered during the calibration process. The material model selection module 216 may, for example, allow the user 102 to toggle which of the available model parameters to use in the calibration process. In some embodiments, the material model selection module 216 may automatically populate a default initial value for one or more of the available model parameters for that selected model. The material model selection module 216 may allow the user 102 to enter initial values for the model parameters or alter the default initial values.

In some embodiments, the material model selection module 216 allows the user 102 to configure minimization-related settings such as, for example, selecting a particular minimization algorithm from a list of available minimization algorithms, tune particular settings of that minimization algorithm, and select an error measure (e.g., from a list of available error measures) that is used as one of the ingredients for the objective function.

The stability configuration module 218, in the example embodiment, allows the user 102 to enable stability consideration during the calibration process and to configure aspects of the stability consideration. The stability configuration module 218 may provide functionality that allows the user 102 to enable or disable stability consideration during the calibration process. When stability consideration is disabled, the calibration computation module 220 calibrates the material model without evaluating the stability of the material model during the iterative process. When stability consideration is enabled, the calibration computation module 220 evaluates one or more stability criteria during the calibration process (e.g., at each iteration) to determine whether the latest model result is stable. Stability consideration is described in greater detail below.

Further, in the example embodiment, the stability configuration module 218 allows the user 102 to identify one or more strain ranges of interest for this calibration. For example, the user 102 may wish to have the material model stable across one or more ranges of strains, and optionally or one or more modes of deformation. As such, the stability configuration module 218 allows the user 102 to enter strain ranges such as, for example, a range of strain in the loading direction and lateral direction, a volume ratio, and a shear strain. Each strain range is associated with a particular type of deformation mode, and may include a lower threshold, and upper threshold, or both. In some embodiments, strain ranges may extend beyond the strain values available in the experimental test data sets. The user 102 may desire a broad strain range of stability since the finite element simulation process may perform material calculations across a broad strain range during a simulation.

Further, the user 102 may restrict the stability consideration to only certain deformation modes. For example, the user 102 may select an option to apply the strain range settings to all deformation modes, or to only uniaxial deformation, or to only biaxial deformation, and so forth.

The calibration computation module 220, in the example embodiment, performs the computations associated with the calibration process. More specifically, the calibration computation module 220 uses a numerical constrained optimization algorithm to minimize an objective function that incorporates the errors between the test data set and the model response, together with one or more constraint criteria, in an iterative process until convergence is reached.

In the example embodiment, the calibration computation module 220 performs an iterative minimization process that uses a user-selected numerical minimization algorithm to calibrate the material model, starting with the initial parameter values. During the minimization process, the calibration computation module 220 uses an objective function to compute an error between the material model with the current parameter value(s) (the "current model response" at this iteration) and the test data set. Different minimization algorithms use their own iterative schemes, but in general, each minimization algorithm evaluates the objective function multiple times during each minimization iteration with the material parameters being repeatedly modified such that, at the end of the iteration, the value of the objective function is less than the value of the objective function at the beginning of the iteration. This iterative process continues until a certain termination criterion is fulfilled (e.g., the change in material properties is below a pre-determined threshold).

During each evaluation of the objective function, after the error between the material model with the current parameter values and the test data sets have been computed, the calibration computation module 220 automatically evaluates stability of the material model.

One constraint criterion that may be used is Drucker's stability criterion, which can be stated mathematically as:

$$d\varepsilon : D : d\varepsilon > 0,$$

where $d\varepsilon$ is the incremental strain and D is a tensor quantity representing the material tangent stiffness. For numerical evaluations, tensor quantities are represented in an equivalent matrix format. The values of components, $D_{ijkl}$, of the material tangent stiffness depends on the selected mathematical material model and the current values of its set of parameters.

Under classic Drucker's stability criterion, a material is considered stable if the incremental work done by incremental (e.g., Kirchoff) stresses along the corresponding incremental displacements is positive. Material models that do not satisfy this stability criterion are considered unstable. This can be expressed as:

$$d\tau_{ij}d\varepsilon_{ij} > 0,$$

where $d\tau_{ij}$ are the incremental stress components and $d\varepsilon_{ij}$ are the incremental logarithmic strain components. Using the constitutive relation:

$$d\tau_{ij} = D_{ijkl}d\varepsilon_{kl},$$

the stability condition can be written as:

$$D_{ijkl}d\tau_{ij}d\varepsilon_{ij} > 0, \tag{1}$$

where $D_{ijkl}$ are the components of material tangent stiffness tensor and are functions of the material properties being calibrated. Using unstable material responses in a numerical (e.g., finite element) simulation is less desirable in many cases because it may lead to non-unique or unphysical solutions.

In this example, the calibration computation module 220 implements an optimization process with stability criterion based on Drucker's stability criterion, but the optimization process enforces the stability criterion during the minimization process, iteratively converging to a set of material parameters that satisfies the stability criterion at the provided strain ranges while minimizing the errors between the test data sets and the corresponding response predicted by the selected material model.

The objective function to be minimized during the optimization process, in the example embodiment, is a mathematical function that provides a measure of error between the recorded test data sets and the corresponding predicted material response at a given iteration, such that the global minimum value of the objective function is achieved for the ideal case when the predicted material response perfectly matches the recorded test data. The objective function may depend on the recorded test data to be used for calibration, or on the current values of material properties (e.g., of the constitutive response chosen to be calibrated). The objective function may be created in different ways (e.g., including based on user choices). In some embodiments, if multiple test data sets are used, a weighting factor for errors may be used between each data set.

Numerous constrained optimization techniques are known and can be used here. In the example embodiment, the objective function used is:

$$\Pi(c) = \sum_{1}^{\# \, test \, data \, sets} W_i E_i(c), \tag{2}$$

where $E_i(c)$ is the value characterizing the errors between the $i^{th}$ test data set and the corresponding predicted response and $W_i$ is the weighting factor for $E_i(c)$, and c is an array of material properties that are identified as design variables (e.g., by the user). In the example embodiment, a mean square error measure may be used in the objective function for $E_i$:

$$E_i(c) = \sum_{k}^{\# \, recorded \, test \, values} \left(\sigma_k^{test} - \sigma_k(c)^{predicted}\right)^2, \tag{3}$$

and, again, the stability condition is Drucker's criterion:

$$D_{ijkl}(c)d\tau_{ij}d\varepsilon_{ij} > 0, \qquad (1)$$

where $D_{ijkl}(c)$ are the components of material tangent stiffness tensor and are functions of the material properties, $c$, being calibrated. This relation is satisfied if $D(c)$ is positive definite.

In the example embodiment, the calibration computation module 220 checks for positive definiteness of $D$ using Sylvester's criterion, which states that a symmetric real matrix is positive definite if all of the leading principal minors of $D$ are positive. For an NxN matrix, Sylvester's criterion leads to a set of N inequality constraints that can be written as:

$$g_i(D) > 0, i = 1 \dots N,$$

where $g_i(D)$ are functions that map various components of $D$ into real scalars. For example, for a 3x3 $D$ matrix, Sylvester's criterion is equivalent to $D$ components satisfying the following three inequalities:

$$g_1(D) = D_{11} + D_{22} + D_{33} > 0, \qquad (4a)$$

$$g_2(D) = D_{11}D_{22} + D_{22}D_{33} + D_{33}D_{11} - D_{23}^2 - D_{13}^2 - D_{12}^2 > 0, \qquad (4b)$$

$$g_3(D) = \det(D) > 0. \qquad (4c)$$

In the example embodiment, such inequalities, (4a) - (4c), are treated as constraints during the calibration process, which pushes the calibration process towards a set of parameters that should satisfy the stability criterion. The constrained optimization procedure can be stated mathematically as:

minimize the objective function, $\Pi(c)$,

subject to: $g_i(D) > 0$, $i = 1 \dots N$.

The above inequality constraint problem can be recast as an equality constraint problem by defining additional variables ($s_i$, $i = 1...N$):

minimize the objective function, $\Pi(c)$,

subject to: $\hat{g}_i(D, s_i) = g_i(D) - s_i^2 = 0$, $i = 1 \dots N$.

Succeeding to obtain a solution that satisfies the stability criterion, in some embodiments, depends on the choice of minimization algorithm and the constraint enforcement method.

In other embodiments, positive definiteness may be determined by performing an eigenvalue analysis of $D$ and checking that all eigenvalues are positive. In still other embodiments, positive definiteness may be determined by performing an LDL factorization of $D$, where if the factorization exists and all of the values of D are positive, then $D$ is a positive definite matrix.

In the example embodiment, the calibration computation module 220 uses a penalty function as a constraint enforcement method during the calibration process. A penalty function maps the constraint violation onto a positive scalar value that is added to the objective function. If a constraint is satisfied, then there is no violation of that constraint and it does not contribute to the objective function. If the constraint is not satisfied, then the violation value is the value by which the constraint is not satisfied (e.g., the value that needs to be added in order to satisfy the constraint). For example, if $D_{11} + D_{22} + D_{33} = 2$, then this constraint is not violated because the value is greater than 0. However, if $D_{11} + D_{22} + D_{33} = -5$, then this constraint is violated, and the violation value is $v = 5$.

In the example embodiment, the objective function is modified with a quadratic penalty function, leading to the following modified objective function, $\Pi^*$:

$$\Pi^* = \Pi + k * \sum_m^{\# \, deformation \, modes} \left( \sum_k^{\# \, violations} v_k^2 \right), \qquad (5)$$

where $k$ is a positive scalar referred to as the penalty stiffness, which can be controlled by the user 102. In one example, the quadratic penalty method includes minimizing the functional:

$$\hat{\pi}(c, \kappa_1, s_1, \kappa_2, s_2, \kappa_3, s_3) = \pi(c) + \frac{1}{2}\kappa_1\big(\hat{g}_1(D, s_1)\big)^2 + \frac{1}{2}\kappa_2\big(\hat{g}_2(D, s_2)\big)^2 + \frac{1}{2}\kappa_3\big(\hat{g}_3(D, s_3)\big)^2$$

In other embodiments, other penalty or barrier functions may be used. One of the advantages of enforcing stability constraints using penalty functions is that they can be used with a wide range of unconstrained minimization algorithms. For example, it is computationally inexpensive to implement and use penalty constraint enforcement with derivative-free minimization algorithms.

In other embodiments, constraint minimization may be performed with Lagrange multipliers. For example, enforcing inequality constraints may be performed by minimizing the functional:

$$\hat{\pi}(c, \lambda_1, s_1, \lambda_2, s_2, \lambda_3, s_3) = \pi(c) + \lambda_1\hat{g}_1(D, s_1) + \lambda_2\hat{g}_2(D, s_2) + \lambda_3\hat{g}_3(D, s_3).$$

In still other embodiments, constraint minimization may be performed with augmented Lagrange multipliers by minimizing the functional:

$$\hat{\pi}(c, \lambda_1, \kappa_1, s_1, \lambda_2, \kappa_2, s_2, \lambda_3, \kappa_3, s_3) = \pi(c) + \lambda_1\hat{g}_1(D, s_1) + \lambda_2\hat{g}_2(D, s_2) + \lambda_3\hat{g}_3(D, s_3)$$

$$\frac{1}{2}\kappa_1\big(\hat{g}_1(D, s_1)\big)^2 + \frac{1}{2}\kappa_2\big(\hat{g}_2(D, s_2)\big)^2 + \frac{1}{2}\kappa_3\big(\hat{g}_3(D, s_3)\big)^2.$$

In still other embodiments, constraint minimization may be performed with augmented perturbed Lagrange multipliers by minimizing the functional:

$$\hat{\pi}(c, \lambda_1, \kappa_1, \epsilon_1, s_1, \lambda_2, \kappa_2, \epsilon_2, s_2, \lambda_3, \kappa_3, \epsilon_3, s_3) = \pi(c) + \lambda_1\hat{g}_1(D, s_1) + \lambda_2\hat{g}_2(D, s_2)$$

$$+ \lambda_3\hat{g}_3(D, s_3) + \frac{1}{2}\kappa_1\big(\hat{g}_1(D, s_1)\big)^2 + \frac{1}{2}\kappa_2\big(\hat{g}_2(D, s_2)\big)^2 + \frac{1}{2}\kappa_3\big(\hat{g}_3(D, s_3)\big)^2$$

$$- \frac{1}{2}\epsilon_1\lambda_1{}^2 - \frac{1}{2}\epsilon_2\lambda_2{}^2 - \frac{1}{2}\epsilon_3\lambda_3{}^2.$$

As such, in the example embodiment, the penalty function is added to the error value included in the objective value each time there are stability violations, and the amount of the penalty values added depends on the values of the stability violations. Accordingly, the application of the penalty function forces the calibration process to modify the values of material parameters in such way that they satisfy the stability criterion.

In the example embodiment, the calibration computation module 220 terminates the iterative process when any one or more of the following are true: (1) the change in solution (e.g., material properties the user 102 is trying to calibrate) between subsequent iterations is smaller than a pre-defined (e.g., user-defined) threshold value; (2) the change in the value of the objective function, including penalty, is less than a pre-defined (e.g., user-defined) threshold value; (3) the total number of occurred objective function evaluations is greater than a pre-defined (e.g., user-defined) maximum allowed number of function evaluations; (4) the total number of the minimization iterations is greater than a pre-defined (e.g., user-defined) value; and (5) the algorithm intercepted an execution error. In some situations, the calibration process is not guaranteed to succeed. As such, the user 102 may have to adjust input parameters and rerun the calibration. In some embodiments, the calibration computation module 220 may detect the failure and automatically adjust one or more parameters and rerun the calibration attempt. In some embodiments, the material calibration module 130 may use sequential quadratic programming (SQP) for constraint optimization.

After the calibration computation module 220 has converged upon a solution, the calibration process terminates with a resultant set of model parameters for the material model. As such, the material model in combination with the resultant model parameters represents a calibrated material model. In the example embodiment, the calibration computation module 220 displays the calibrated model parameters to the user 102, and may further plot the calibrated material model (e.g., plotting values of the resultant material model over the test sets) via graphing module 212. As such, the user 102 may visually review both the resultant material model parameters as well as a plot of the calibrated material model across the specified strain ranges.

The simulation interface module 222, in the example embodiment, saves the calibrated material model within the database 116 and provides the calibrated material model for use with the simulation software system 120. The simulation interface module 222 may allow the user 102 to export the calibrated material model for use with another simulation system 100. The calibrated material model may be assigned to particular components (e.g., mesh regions) of simulation models and used to approximate response of the assigned components during those simulations.

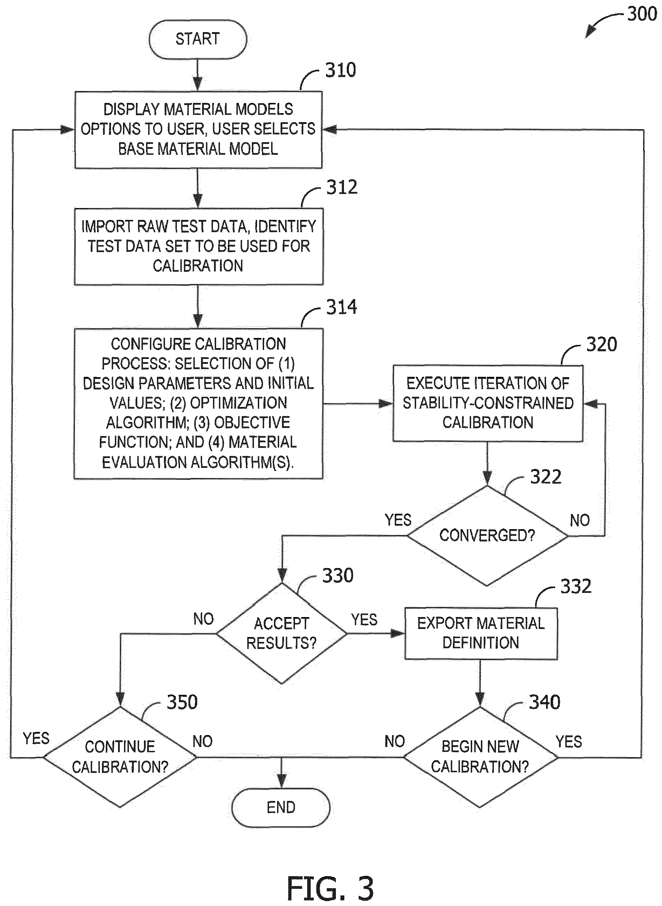

FIG. 3 is a flow chart of an example calibration process 300 for generating material models using stability-based constrained numerical calibration. In the example embodiment, the calibration process 300 is performed by the material calibration module 130 executing on the computing device 110 shown in FIG. 1. The process 300 includes displaying a list of material model options to the user 102, allowing the user 102 to select a base material model for use in the process 300 (see operation 310). The material calibration module 130 may display a list of model types from which the user 102 may select, after which the material calibration module 130 may display a list of base material models associated with the selected type.

In the example embodiment, the process 300 also includes importing raw test data for use in the calibration process 300 (see operation 312). The material calibration module 130 may display the raw test data to the user 102, allowing the user 102 to configure the test data set (e.g., selecting which data pairs to use, identify units of measure for the columns, deformation type associated with the test data set, and so forth).

The process 300 further includes additional configuration of the calibration process 300 (see operation 314). The additional configuration may include, for example, allowing the user 102 to select which design parameters to use of those available for the selected material model, automatically populating initial values for the design parameters, allowing the user 102 to enter or alter those design parameters. The calibration configuration also includes identifying an optimization algorithm associated with the selected material model. Further, the calibration configuration includes identifying an objective function for use in evaluating the error at each iteration, as well as a convergence criterion for exiting the iterative process. The calibration configuration also includes allowing the user 102 to enable stability-constrained calibration, as well as associated configuration parameters such as stiffness, penalty function, and strain ranges.

In the example embodiment, the process 300 also includes iteratively executing the stability-constrained calibration using the configured features. More specifically, the material calibration module 130 executes a first iteration of the stability-constrained calibration, evaluating the optimization algorithm based on the initial values of the design parameters to generate model results for that iteration. The material calibration module 130 then evaluates the model results for that iteration against the objective function with the penalty function to determine whether the model results have produced an error below a pre-determined convergence value. Each minimization algorithm has a specific iterative scheme which may significantly differ between algorithms. In the example embodiment, at each iteration, the objective function is evaluated a certain number of times. During each iteration, the minimization algorithm adjusts the solution in such way that the objective function is decreased (e.g., assuming a minimization process). This approach is also specific to each algorithm. If any constraints are violated, a penalty value is generated and added to the objective function during its evaluation. Hence, the minimization algorithm moves the solution away from values that trigger constraint violations.

In the example embodiment, violations of the stability criterion are evaluated at a number of strain values within the strain range during penalty computation at each iteration. For example, if the user 102 selects a strain range of [0,1] and a strain increment of 0.01, the material calibration module 130 checks for stability violations at strain values of 0.00, 0.01, 0.02, ..., 0.99, and 1.00 during the evaluation of the penalty function. Any stability violation will result in an increased penalty value which in turn will increase the value of the objective function.

FIG. 4 is an illustration of an example view 400 of a graphical user interface provided by the material calibration module 130 during a calibration process without stability constraint. In the example embodiment, the graphical user interface includes a material model panel 410 within which the user 102 has selected a hyperelastic Ogden material model for calibration. The material model panel 410 includes a list of design parameters 412 provided by the model, check boxes indicating which design parameters 412 the user 102 has elected to use during this calibration, as well as display boxes for associated parameter values 414. In this example, Ogden includes design parameters mu1, alpha1, mu2, alpha2, mu3, alpha3, D1, D2, and D3, and the user 102 has elected to use all design parameters except D1, D2, and D3. Further, at this stage in this example, an unconstrained calibration has already been executed using the selected parameters and, as such, the values 414 displayed here are calibrated values generated by the calibration process, though without stability constraint.

In the example embodiment, the user 102 has loaded a single test data set for uniaxial tension, as exhibited in a test datasets panel 420. In a graph panel 430, samples from the test data set are plotted in dots on the graph, which plots nominal strain on the x-axis and nominal stress on the y-axis. Further, the graph illustrates a line plot of the calibrated model (e.g., based on the resultant parameter values 414). A calibration history panel 450 is also provided, illustrating an objective value plotted for each iteration of the calibration process, showing the calibration process converging to a deviatoric of 0.991.

The graphical user interface also includes an optimization controls panel that includes a material stability settings pane 440. The material stability settings pane 440, in this example, shows "include stability computation in calibration" turned on (e.g., checked) but a stability stiffness penalty set to 0. As such, no penalty for stability consideration was imposed during the calibration process. As such, and as mentioned above, the calibration process in this example was unconstrained with regard to stability.

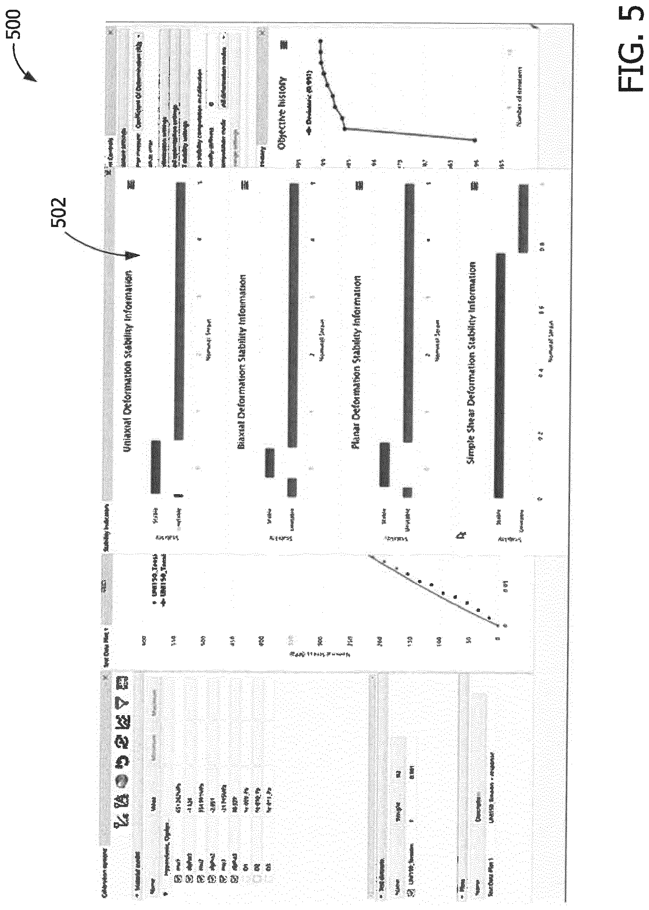

FIG. 5 is an illustration of another example view 500 of the graphical user interface shown in FIG. 4 that includes stability indicators panel 502 provided by the material calibration module 130 during the unconstrained calibration process. In the example embodiment, the stability indicators panel 502 shows bar graph pairs for each of several deformation modes, showing over what strain ranges the resultant calibrated material model of FIG. 4 is stable or unstable. In this example, the calibrated material model is unstable large strain ranges for uniaxial, biaxial, planar and simple shear deformations.

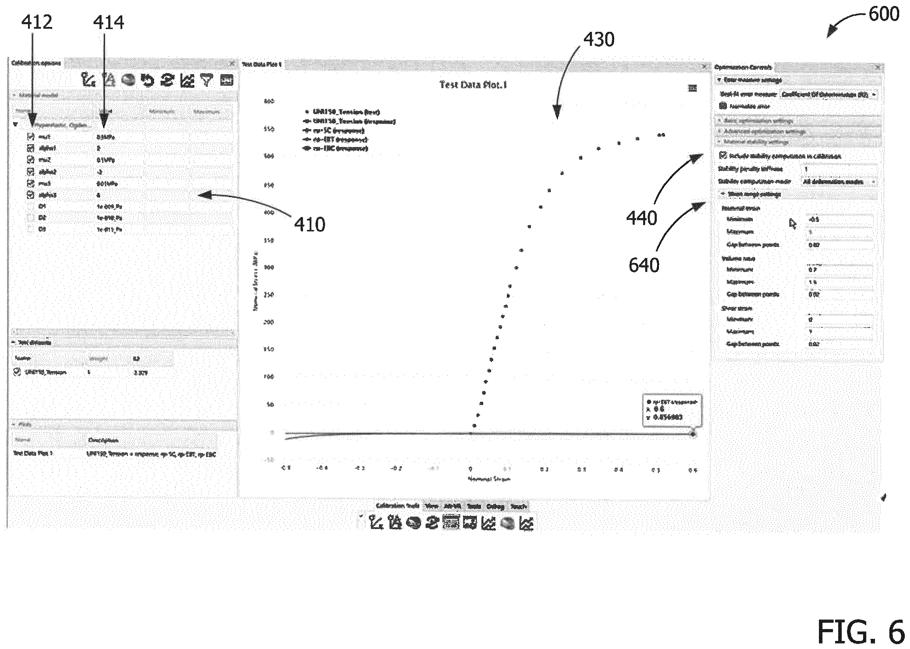

FIG. 6 is an illustration of an example view 600 of a graphical user interface provided by the material calibration module 130 during a calibration process that includes stability constraint consideration as described herein. In the example embodiment, the graphical user interface includes the material model panel 410 within which the user 102 has selected a hyperelastic Ogden material model for calibration, similar to the example of FIG. 4. In this example, the values 414 displayed here are initial values, either default values automatically populated by the materials calibration module 130 or input by the user 102, and the user 102 has not yet executed the calibration process. As such, the graph includes the dots of the test data arcing up across the center of the graph, while a plot of the uncalibrated material model is shown as lines across the x-axis. Further, in the example embodiment, the user 102 has expanded a strain range settings panel 640 of the material stability settings panel 440 and provided several range settings with which to focus the stability of the calibration process (e.g., minimum and maximum values for nominal strain, volume ratio, and shear strain).

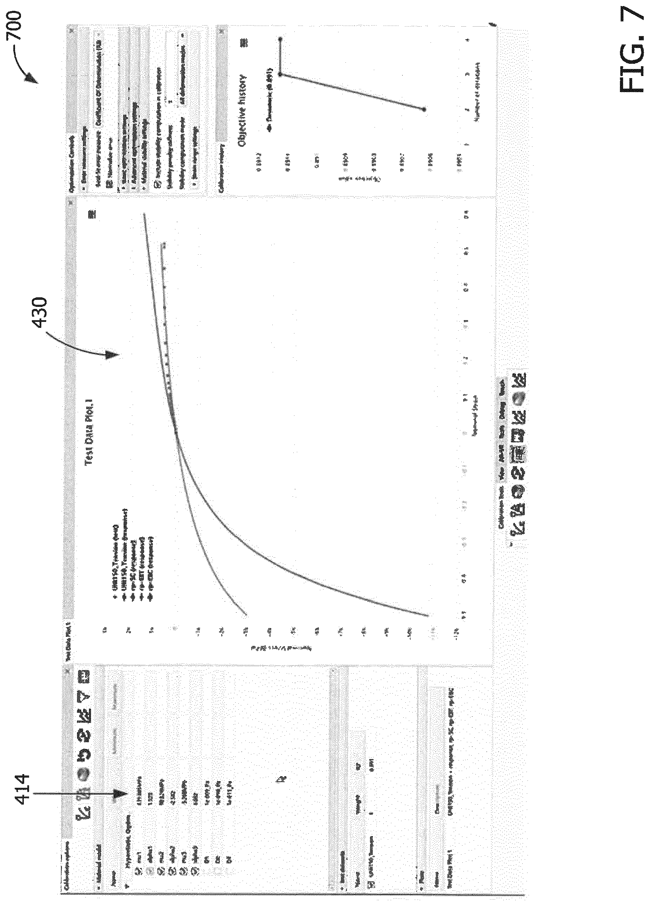

FIG. 7 illustrates a view 700 of the example shown in FIG. 6 after the user 102 has executed the stability-constrained calibration process. In this example, the material calibration module 130 has generated a stability-constrained calibrated material model, represented here by the line plots.

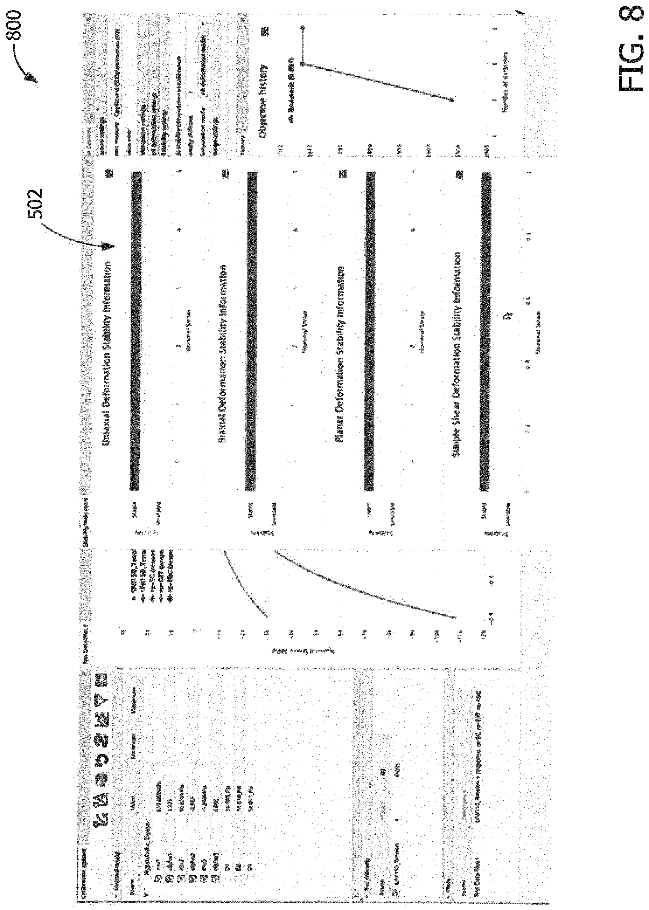

FIG. 8 is an illustration of another example view 800 of the graphical user interface shown in FIG. 7 that includes the stability indicators panel 502 after the stability-constrained calibration process. In the example embodiment, the stability indicators panel 502 shows bar graph pairs for each of several deformation modes, showing over what strain ranges the resultant calibrated material model of FIG. 4 is stable or unstable. In this example, the calibrated material model is stable across the entire strain ranges specified by the user 102, and for each mode of deformation.

As will be appreciated based on the foregoing specification, the above-described embodiments of the disclosure may be implemented using computer programming or engineering techniques including computer software, firmware, hardware or any combination or subset thereof, wherein the technical effect is a system for generating stable material models and using such stable material models in computer simulations. Any such resulting program, having computer-readable code means, may be embodied or provided within one or more computer-readable media, thereby making a computer program product, i.e., an article of manufacture, according to the discussed embodiments of the disclosure. The computer-readable media may be, for example, but is not limited to, a fixed (hard) drive, diskette, optical disk, magnetic tape, semiconductor memory such as read-only memory (ROM), and/or any transmitting/receiving medium such as the Internet or other communication network or link. The article of manufacture containing the computer code may be made and/or used by executing the code directly from one medium, by copying the code from one medium to another medium, or by transmitting the code over a network.

These computer programs (also known as programs, software, software applications, "apps", or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The "machine-readable medium" and "computer-readable medium," however, do not include transitory signals. The term "machine-readable signal" refers to any signal used to provide machine instructions and/or data to a programmable processor.

At least one of the technical problems addressed by this system includes: (i) avoiding generation and use of material models that may be unstable in certain situations; and (ii) need for manual intervention and testing to determine when a material model may be unstable. Other technical problems addressed by the system and methods described herein may include increased computer processing due to unnecessary components appearing in the system, thus slowing down the computer.

The methods and systems described herein may be implemented using computer programming or engineering techniques including computer software, firmware, hardware, or any combination or subset thereof, wherein the technical effects may be achieved by performing at least one of the following steps: (a) displaying to a user a graphical user interface configured to allow the user to import the experimental test data set; (b) receive an identification of the test data set for material model calibration; (c) identifying a material model, the material model includes one or more parameters of a parameter set to be calibrated during the material model calibration, the parameter set starting with a set of initial parameter values; (d) performing an iterative optimization process to calibrate the material model, the iterative optimization process uses an optimization algorithm that enforces a constraint based on Drucker's stability criterion across one or more predetermined strain ranges; (e) terminating the iterative optimization process, the optimization process generates a calibrated material model; and (f) assigning the calibrated material model to a component of a simulation model based on input from the user, a real-world equivalent of the component being made of the physical material; and

The resulting technical effect achieved by this system is at least one of reducing computational requirements for generating stable material models, generating models that are stable over particular ranges, and reducing computational waste from performing failed simulations that previously relied upon unstable material models.

This written description uses examples to disclose the disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

**Claims**

1. A computer simulation system comprising:

   a memory storing an experimental test data set, the experimental test data set includes experimentally-obtained stress-strain data for a sample of a physical material subjected to one or more deformation modes during a real-world experiment; and
   a processor configured to execute instructions stored in the memory, which, when executed by the processor, cause the processor to at least:

   display to a user a graphical user interface configured to allow the user to import the experimental test data set;
   receive an identification of the test data set for material model calibration;
   identify a material model, the material model includes one or more parameters of a parameter set to be calibrated during the material model calibration, the parameter set starting with a set of initial parameter values;
   perform an iterative optimization process to calibrate the material model, the iterative optimization process uses an optimization algorithm that enforces a constraint based on Drucker's stability criterion across one or more predetermined strain ranges;
   terminate the iterative optimization process, the optimization process generates a calibrated material model;
   assign the calibrated material model to a component of a simulation model based on input from the user, a real-world equivalent of the component being made of the physical material; and
   perform a simulation that includes the component, the simulation using the stable material model and stable set of parameters to simulate response of the real-world equivalent during the simulation.

2. The computer simulation system of claim 1, wherein the instructions further cause the processor to:

   display a stability calibration pane within the graphical user interface, the stability calibration pane allows the user to enable or disable the stability constraint violation determination during the iterative process; and/or
   display a stability calibration pane within the graphical user interface, the stability calibration pane allows the user to identify a range for use during the stability constraint violation determination, the range identifies a range of values within which the material model is to be evaluated for stability.

3. The computer simulation system of claims 1 or 2, wherein the graphical user interface further allows the user to select the experimental test data set as a subset of the imported data.

4. The computer simulation system of any one of claims 1 to 3, wherein the parameter set has a current set of parameter values at each iteration, wherein each iteration of the iterative optimization process includes:

> evaluating a response of the material model at the current set of parameter values with an objective function, thereby generating an error between the material model response and the test data set;
> computing an updated set of parameter values for the material model based on the error;
> determining that the material model with the updated set of parameters violates a stability constraint;
> upon determining the stability constraint violation, applying a penalty function to the objective function to generate a modified objective function to be used at the next inner iteration; and
> terminating the constraint violation process for the current iteration of the optimization process when the material model with the updated set of parameters does not violate the stability constraint.

5. The computer simulation system of claim 4, wherein determining that the material model with the updated set of parameters violates the stability constraint includes determining that a material stiffness matrix of the material model is positive definite, and wherein determining that a material stiffness matrix of the material model is positive definite optionally includes using Sylvester's criterion.

6. A method of calibrating a material model for use in a computer simulation, the method is performed by a processor with a memory, the method comprising:

> storing, in the memory, an experimental test data set, the experimental test data set includes experimentally-obtained stress-strain data for a sample of a physical material subjected to one or more deformation modes during a real-world experiment;
> displaying to a user a graphical user interface configured to allow the user to import the experimental test data set;
> receiving an identification of the test data set for material model calibration;
> identifying a material model, the material model includes one or more parameters of a parameter set to be calibrated during the material model calibration, the parameter set starting with a set of initial parameter values;
> performing an iterative optimization process to calibrate the material model, the iterative optimization process uses an optimization algorithm that enforces a constraint based on Drucker's stability criterion across one or more predetermined strain ranges;
> terminating the iterative process, the iterative process generates a calibrated material model;
> assigning the calibrated material model to a component of a simulation model based on input from the user, a real-world equivalent of the component being made of the physical material; and
> performing a simulation that includes the component, the simulation using the stable material model and stable set of parameters to simulate response of the real-world equivalent during the simulation.

7. The method of claim 6, further comprising:

> displaying a stability calibration pane within the graphical user interface, the stability calibration pane allows the user to enable or disable the stability constraint violation determination during the iterative process; and or
> displaying a stability calibration pane within the graphical user interface, the stability calibration pane allows the user to identify a range for use during the stability constraint violation determination, the range identifies a range of values within which the material model is to be evaluated for stability.

8. The method of claims 6 or 7, wherein the graphical user interface further allows the user to select the experimental test data set as a subset of the imported data.

9. The method of any one of claims 6 to 8, wherein the parameter set has a current set of parameter values at each iteration, wherein each iteration of the iterative optimization process includes:

> evaluating a response of the material model at the current set of parameter values with an objective function, thereby generating an error between the material model response and the test data set;
> computing an updated set of parameter values for the material model based on the error;
> determining that the material model with the updated set of parameters violates a stability constraint;
> upon determining the stability constraint violation, applying a penalty function to the objective function to generate a modified objective function to be used at the next inner iteration; and
> terminating the constraint violation process for the current iteration of the optimization process when the material model with the updated set of parameters does not violate the stability constraint.

**10.** The method of claim 9, wherein determining that the material model with the updated set of parameters violates the stability constraint includes determining that a material stiffness matrix of the material model is positive definite, and wherein determining that a material stiffness matrix of the material model is positive definite optionally includes using Sylvester's criterion.

**11.** A computer-readable storage media having computer-executable instructions embodied thereon, wherein, when executed by at least one processor, the computer-executable instructions cause the processor to:

store, in the memory, an experimental test data set, the experimental test data set includes experimentally-obtained stress-strain data for a sample of a physical material subjected to one or more deformation modes during a real-world experiment;
display to a user a graphical user interface configured to allow the user to import the experimental test data set;
receive an identification of the test data set for material model calibration;
identify a material model, the material model includes one or more parameters of a parameter set to be calibrated during the material model calibration, the parameter set starting with a set of initial parameter values;
perform an iterative optimization process to calibrate the material model, the iterative optimization process uses an optimization algorithm that enforces a constraint based on Drucker's stability criterion across one or more predetermined strain ranges;
terminate the iterative process, the iterative process generates a calibrated material model;
assign the calibrated material model to a component of a simulation model based on input from the user, a real-world equivalent of the component being made of the physical material; and
perform a simulation that includes the component, the simulation using the stable material model and stable set of parameters to simulate response of the real-world equivalent during the simulation.

**12.** The computer-readable medium of claim 11, wherein the computer-executable instructions further cause the processor to:
display a stability calibration pane within the graphical user interface, the stability calibration pane allows the user to enable or disable the stability constraint violation determination during the iterative process.

**13.** The computer-readable medium of claims 11 or 12, wherein the computer-executable instructions further cause the processor to:
display a stability calibration pane within the graphical user interface, the stability calibration pane allows the user to identify a range for use during the stability constraint violation determination, the range identifies a range of values within which the material model is to be evaluated for stability.

**14.** The computer-readable medium of any one of claims 11 to 13, wherein the parameter set has a current set of parameter values at each iteration, wherein each iteration of the iterative optimization process includes:

evaluating a response of the material model at the current set of parameter values with an objective function, thereby generating an error between the material model response and the test data set;
computing an updated set of parameter values for the material model based on the error;
determining that the material model with the updated set of parameters violates a stability constraint;
upon determining the stability constraint violation, applying a penalty function to the objective function to generate a modified objective function to be used at the next inner iteration; and
terminating the constraint violation process for the current iteration of the optimization process when the material model with the updated set of parameters does not violate the stability constraint.

**15.** The computer-readable medium of claim 14, wherein determining that the material model with the updated set of parameters violates the stability constraint includes determining that a material stiffness matrix of the material model is positive definite, and wherein optionally determining that a material stiffness matrix of the material model is positive definite includes using Sylvester's criterion.

**FIG. 1**

200 ⌐

130 ⌐

MATERIAL CALIBRATION MODULE

⌐ 210
GRAPHICAL USER
INTERFACE MODULE

⌐ 212
GRAPHING
MODULE

⌐ 214
TEST DATA
MODULE

⌐ 216
MATERIAL MODEL
SELECTION MODULE

⌐ 218
STABILITY
CONFIGURATION
MODULE

⌐ 220
CALIBRATION
COMPUTATION
MODULE

⌐ 222
SIMULATION
INTERFACE
MODULE

# FIG. 2

FIG. 3

FIG. 4

FIG. 5

FIG. 6

EP 3 674 956 A1

FIG. 7

FIG. 8

Europäisches
Patentamt

European
Patent Office

Office européen
des brevets

**EUROPEAN SEARCH REPORT**

Application Number

EP 19 21 1978

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/010427 A1 (HOFFMAN EDWARD L [US]) 12 January 2006 (2006-01-12) * paragraph [0002] - paragraph [0100] * * figures 1-9 * ----- | 1-15 | INV. G06F30/10 G06F30/17 G06F30/20 |
| X | US 9 977 841 B1 (ASSAKER ROGER [BE] ET AL) 22 May 2018 (2018-05-22) * column 1 - column 10 * * figures 1-5 * ----- | 1-15 | |

TECHNICAL FIELDS
SEARCHED      (IPC)

G06F

The present search report has been drawn up for all claims

| Place of search | Date of completion of the search | Examiner |
|---|---|---|
| Munich | 13 May 2020 | Rackl, Günther |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 21 1978

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2020

| Patent document cited in search report | Publication date | Patent family member(s) | Publication date |
|---|---|---|---|
| US 2006010427 A1 | 12-01-2006 | NONE | |
| US 9977841 B1 | 22-05-2018 | NONE | |

EPO FORM P0459

For more details about this annex : see Official Journal of the European Patent Office, No. 12/82